# EUROPEAN PATENT APPLICATION

(11) **EP 1 496 054 A1**
(43) Date of publication of application: **12.01.2005**
(21) Application number: 03725609.6
(22) Date of filing: 18.04.2003
(51) Int. Cl.: C07D 307/79, A61K 31/343, A61P 3/10, A61P 9/00, A61P 9/10, A61P 9/12

(54) **MEDICINAL COMPOSITION FOR IMPROVING ANGIOTENSION-REGULATING FUNCTION OF BLOOD VESSEL ENDOTHELIUM**

(30) Priority: 18.04.2002 JP 2002116419
(71) Applicant: CHUGAI SEIYAKU KABUSHIKI KAISHA, Tokyo, 115-8543 (JP)
(72) Inventor: CYNSHI, Osamu c/o Chugai Seiyaku Kabushiki Kaisha, Gotenba-shi, Shizuoka 412-8513 (JP); KAWABE, Yoshiki, c/o Chugai Seiyaku K. K., Gotenba-shi, Shizuoka 412-8513 (JP); KOMORI, Toshihiko, c/o Chugai Seiyaku K. K., Chuo-ku, Tokyo 104-8301 (JP)
(74) Representative: VOSSIUS & PARTNER
(86) International application number: PCT/JP2003/004987
(87) International publication number: WO 2003/087080

(57) **Abstract**

Herein provided is a novel pharmaceutical composition for ameliorating the vascular tone-regulating function of vascular endothelium, comprising a compound represented by formula (1): 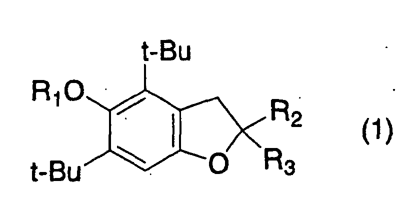 wherein
R₁ represents a hydrogen atom or an acyl or arylalkyloxycarbonyl group; R₂ and R₃ are identical or different and represent an optionally substituted alkyl group, an optionally substituted alkenyl group or an optionally substituted alkynyl group; or R₂ and R₃ may together form a cycloalkyl group.

## Description

### FIELD OF THE INVENTION

The present invention relates to a pharmaceutical composition and a method for ameliorating the vascular tone-regulating function of vascular endothelium, and a use of certain compounds for the manufacture of such a composition. More specifically, the present invention relates to a pharmaceutical composition and a method for ameliorating the impairment of the vascular tone-regulating function of vascular endothelium induced by a disease of adults, such as, hypertension, diabetes or arteriosclerosis obliterans.

### BACKGROUND ART

With growing an aging society, the incidence of the diseases of adults such as hypertension, diabetes, arteriosclerosis and arteriosclerosis obliterans is continuing to increase year by year. A common feature of these diseases of adults is continuous progress of vascular lesions with the development of the diseases. The vascular lesions are mildly exacerbated with an increased risk of developing serious diseases such as myocardial infarction. In the process of the progress of vascular lesions, vascular endothelial dysfunction induced by damaged vascular endothelial cells is especially noteworthy.

Vascular endothelial cells are cells lining the inside of blood vessels. Recent evidence has shown that these cells not only selectively control mass transportation into blood vessels but also play an important role in regulating blood pressure or preventing blood clotting. Recent evidence has also shown that the presence of endothelium-derived relaxing factor (EDRF) secreted from endothelial cells is important for the vascular tone-regulating function of vascular endothelium. If oxygen consumption in a tissue rises as a result of exercise or the like, for example, oxygen supply to the tissue must be increased, and this is achieved by lessening vascular tone in the tissue, i.e., relaxing the vascular endothelium to increase arterial blood influx. In such cases, EDRF seems to be secreted in large quantities from vascular endothelial cells, thereby promoting relaxation of the vascular endothelium.

Accordingly, if vascular endothelial cells are damaged by a disease of adults such as hypertension or diabetes and their EDRF-secreting ability decreases, the vascular tone-regulating function of the vascular endothelium will be impaired. As a result, hypertension, diabetes, arteriosclerosis, arteriosclerosis obliterans or the like progresses, and more blood vessels remain contracted and cannot be relaxed, which causes chronic stenosis or occlusion leading to myocardial infarction or the like. A typical clinical condition induced by the chronic stenosis or occlusion in peripheral arteries is intermittent claudication. The decrease of the EDRF-secreting ability also plays an important role in pathological physiology of vascular lesions other than intermittent claudication. This is evident from reports of the decrease of the EDRF-secreting ability in various diseases of adults. Therefore, if the decrease of the EDRF-secreting ability of vascular endothelial cells is prevented, myocardial infarction and intermittent claudication and other vascular lesions associated with the progress of hypertension, diabetes, arteriosclerosis, arteriosclerosis obliterans or the like will be prevented or treated.

Among drugs reported to prevent the decrease of the EDRF-secreting ability of vascular endothelial cells is probucol, which is known as a drug for treating hyperlipemia. However, administration of probucol lowers high-density lipoprotein cholesterol (HDL-C) and prolongs electrocardiographic QT intervals. These adverse side effects are thought to cause ischemic heart diseases and sudden death, and, therefore, it would be desirable to develop a drug preventing the decrease of the EDRF-secreting ability of vascular endothelial cells without such adverse side effects, and eventually a drug for ameliorating the vascular tone-regulating function of the vascular endothelium.

### DISCLOSURE OF THE INVENTION

An object of the present invention is to provide a novel pharmaceutical composition for ameliorating the vascular tone-regulating function of vascular endothelium.

As a result of extensive research to solve the problems described above, we found that certain dihydrobenzofuran compounds can ameliorate the vascular tone-regulating function of the vascular endothelium.

Accordingly, the present invention provides a pharmaceutical composition for ameliorating the vascular tone-regulating function of vascular endothelium, comprising a compound represented by formula (1): 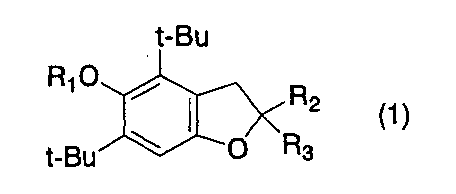 wherein
R₁ represents a hydrogen atom, an acyl or an arylalkyloxycarbonyl group;
R₂ and R₃ are identical or different and represent an optionally substituted alkyl group, an optionally substituted alkenyl group or an optionally substituted alkynyl group; or
R₂ and R₃ may together form a cycloalkyl group;
or a pharmaceutically acceptable salt thereof as an active ingredient.

The present invention also provides a method for ameliorating the vascular tone-regulating function of vascular endothelium, comprising administering to a patient in need thereof, an effective amount of a compound represented by formula (1) or a pharmaceutically acceptable salt thereof.

The present invention also provides a use of a compound represented by formula (1) or a pharmaceutically acceptable salt thereof in the manufacture of a pharmaceutical composition for ameliorating the vascular tone-regulating function of vascular endothelium.

In a preferred embodiment, the compound represented by formula (1) is 4,6-di-t-butyl-5-hydroxy-2,2-di-n-butyl-2,3-dihydrobenzofuran, 4,6-di-t-butyl-5-hydroxy-2,2-di-n-pentyl-2,3-dihydrobenzofuran, 4,6-di-t-butyl-5-hydroxy-2,2-di-n-hexyl-2,3-dihydrobenzofuran, or 4,6-di-t-butyl-5-hydroxy-2,2-di-n-heptyl-2,3-dihydrobenzofuran.

In a preferred embodiment, a condition requiring the amelioration of the vascular tone-regulating function of vascular endothelium is induced by a disease selected from the group consisting of hypertension, diabetes and arteriosclerosis obliterans.

In a more preferred embodiment, a condition requiring the amelioration of the vascular tone-regulating function of vascular endothelium is intermittent claudication.

### BRIEF DESCRIPTION OF THE DRAWINGS

Figure 1 is a graph showing vascular endothelium-dependent relaxation response to acetylcholine using cross-section samples of rat vessels damaged by oxidized LDL after immersion in a 20 µM solution of a test compound.
Figure 2 is a graph showing vascular endothelium-dependent relaxation response to acetylcholine using cross-section samples of rat vessels damaged by oxidized LDL after immersion in a 50 µM solution of a test compound.

### THE MOST PREFERRED EMBODIMENTS OF THE INVENTION

The compounds of formula (1) of the present invention are known. JP 6-206842 A and WO94/08930 describe that these compounds have an antioxidant effect. JP 11-21238 A and WO98/50025 describe that these compounds inhibit atherosclerotic lesions and xanthomatosis. However, it has not been known that the compounds of formula (1) of the present invention ameliorate vascular tone-regulating function.

The compounds of formula (1) used in the present invention can be synthesized by a process described in JP 6-206842 A and corresponding US Patent No. 5,574,178 or European Patent No. 0665208.

In formula (1) of the present invention, examples of the acyl group for R₁ include aliphatic acyl groups having 1-10 carbon atoms and aromatic acyl groups having 7-10 carbon atoms. Preferred specific examples of the aliphatic acyl groups include formyl, acetyl, propionyl and hexanoyl groups, and specific examples of the aromatic acyl groups include benzoyl group. The acyl group is preferably an aliphatic acyl group, more preferably an aliphatic acyl group having 1-6 carbon atoms, especially acetyl group. Examples of the arylalkyloxycarbonyl group for R₁ include those having 7-12 carbon atoms. Preferred examples include those having 7-11 carbon atoms, and more preferred specific examples include benzyloxycarbonyl and naphthylmethoxycarbonyl groups.

R₁ preferably represents a hydrogen atom or an acyl group, more preferably a hydrogen atom or an acetyl group, especially a hydrogen atom.

The alkyl group for R₂ and R₃ means to include straight or branched alkyl groups having 1-20 carbon atoms. Specific examples include methyl, ethyl, n-propyl, isopropyl, n-butyl, isobutyl, s-butyl, t-butyl, n-pentyl, isopentyl, sec-pentyl, t-pentyl, neopentyl, n-hexyl, isohexyl, ethylbutyl, n-heptyl, isoheptyl, ethylpentyl, n-octyl, ethylhexyl, propylpentyl, nonyl, decyl, pentadecyl and stearyl groups. The alkyl group is preferably a straight or branched alkyl group having 1-10 carbon atoms, more preferably a straight alkyl group having 3-8 carbon atoms.

The alkenyl group for R₂ and R₃ means to include straight or branched alkenyl groups having 2-20 carbon atoms. Specific examples include ethenyl, propenyl, isopropenyl, butenyl, isobutenyl, pentenyl, isopentenyl, hexenyl, isohexenyl, ethylbutenyl, heptenyl, isoheptenyl, ethylpentenyl, octenyl, nonenyl, decenyl and pentadecenyl groups. The alkenyl group is preferably a straight or branched alkenyl group having 2-10 carbon atoms, more preferably a straight alkenyl group having 3-8 carbon atoms.

The alkynyl group for R₂ and R₃ means to include straight or branched alkynyl groups having 2-20 carbon atoms. Specific examples include ethynyl, propynyl, isopropynyl, butynyl, isobutynyl, pentynyl, isopentynyl, hexynyl, isohexynyl, ethylbutynyl, heptynyl, isoheptynyl, ethylpentynyl, octynyl, nonynyl, decynyl and pentadecynyl groups. The alkynyl group is preferably a straight or branched alkynyl group having 2-10 carbon atoms, more preferably a straight alkynyl group having 3-8 carbon atoms.

The cycloalkyl group formed by R₂ and R₃ means to include cycloalkyl groups having 3-10 carbon atoms. Specific examples include cyclopropyl, cyclobutyl, cyclopentyl, cyclohexyl, cycloheptyl, cyclooctyl, cyclononyl and cyclodecyl groups. The cycloalkyl group is preferably a cycloalkyl group having 5-8 carbon atoms, more preferably a cycloalkyl group having 4-7 carbon atoms.

Substituents for the optionally substituted alkyl group, optionally substituted alkenyl group and optionally substituted alkynyl group for R₂ and R₃ include, for example, halogen, lower alkyl, lower alkenyl, lower alkynyl, hydroxy, amino, mono- or di-alkylamino, carboxy, acyl, cyano, alkoxy, aryloxy, nitro, halogenoalkyl, aryl and heteroaryl groups, preferably halogen, lower alkoxy, hydroxy, amino, nitro and trifluoromethyl groups.

Especially preferred R₂ and R₃ are straight unsubstituted alkyl groups having 3-8 carbon atoms, specifically n-butyl, n-pentyl, n-hexyl, n-heptyl or n-octyl group, and especially R₂ and R₃ both represent n-pentyl group.

A pharmaceutically acceptable salt of a compound represented by formula (1) of the present invention can be formed when the compound of formula (1) has a group capable of forming an addition salt with an acid or a base in R₂ or R₃. Examples of an acid used to form an acid addition salt include, for example, inorganic acids such as hydrochloric acid, hydrobromic acid, nitric acid, sulfuric acid and phosphoric acid; and organic acids such as acetic acid, lactic acid, oxalic acid, glycolic acid, tartaric acid, malic acid and citric acid. Examples of a base used to form a base addition salt include methylamine, ethylamine, ethanolamine, pyridine, piperidine, morpholine and triethylamine.

Preferred specific compounds represented by formula (1) of the present invention are as follows:
4,6-di-t-butyl-5-hydroxy-2,2-dimethyl-2,3-dihydrobenzofuran;
4,6-di-t-butyl-5-hydroxy-2,2-diethyl-2,3-dihydrobenzofuran;
4,6-di-t-butyl-5-hydroxy-2,2-di-n-propyl-2,3-dihydrobenzofuran;
4,6-di-t-butyl-5-hydroxy-2,2-di-isopropyl-2,3-dihydrobenzofuran;
4,6-di-t-butyl-5-hydroxy-2,2-di-n-butyl-2,3-dihydrobenzofuran;
4,6-di-t-butyl-5-hydroxy-2,2-di-s-butyl-2,3-dihydrobenzofuran;
4,6-di-t-butyl-5-hydroxy-2,2-di-t-butyl-2,3-dihydrobenzofuran;
4,6-di-t-butyl-5-hydroxy-2,2-di-n-pentyl-2,3-dihydrobenzofuran;
4,6-di-t-butyl-5-hydroxy-2,2-di-isopentyl-2,3-dihydrobenzofuran;
4,6-di-t-butyl-5-hydroxy-2,2-di-t-pentyl-2,3-dihydrobenzofuran;
4,6-di-t-butyl-5-hydroxy-2,2-di-neopentyl-2,3-dihydrbbenzofuran;
4,6-di-t-butyl-5-hydroxy-2,2-di-n-hexyl-2,3-dihydrobenzofuran;
4,6-di-t-butyl-5-hydroxy-2,2-di-n-heptyl-2,3-dihydrobenzofuran;
4,6-di-t-butyl-5-hydroxy-2,2-di-n-octyl-2,3-dihydrobenzofuran;
4,6-di-t-butyl-5-hydroxy-2,2-di-n-nonyl-2,3-dihydrobenzofuran; and
4,6-di-t-butyl-5-hydroxy-2,2-di-n-decyl-2,3-dihydrobenzofuran.

An especially preferred specific compound represented by formula (1) is 4,6-di-t-butyl-5-hydroxy-2,2-di-n-pentyl-2,3-dihydrobenzofuran.

The compounds represented by formula (1) of the present invention are useful for treating the impairment of the vascular tone-regulating function of vascular endothelium associated with damaged vascular endothelial cells induced by a disease of adults such as, e.g. hypertension, diabetes or arteriosclerosis obliterans, because they exhibit an excellent ameliorating effect on the impaired vascular tone-regulating function. Without wishing to be bound by any theory, this ameliorating effect on the vascular tone-regulating function seems to result from the capability of the compounds of formula (1) to prevent the decrease of the EDRF-secreting ability of vascular endothelial cells. Conditions with impaired vascular tone-regulating function of vascular endothelium include intermittent claudication induced by chronic vascular constriction or occlusion especially in lower extremity arteries.

The pharmaceutical composition of the present invention can be formulated into various dosage forms by combining a compound represented by formula (1) as an active ingredient with a physiologically acceptable solid or liquid carrier, depending on the administration route. Suitable administration routes include oral administration, parenteral administration such as intravenous injection, sustained-release administration using a sustained-release formulation, and topical administration using a catheter. Examples of the pharmaceutical carrier include those commonly used, such as excipients, binders, disintegrants, lubricants, coating agents, solubilizers, emulsifiers, suspending agents, stabilizers, fats and oils, and solvents. Examples of dosage form include, for example, those suitable for oral; for parenteral, e.g., intravenous, intramuscular or subcutaneous; for buccal; for nasal; or for rectal administration or for inhalation or insufflation administration. Specifically, dosage forms include tablets, pills, capsules, granules, solutions, syrups, suspensions, emulsions, injections and aerosols.

The dose of the compound represented by formula (1) of the present invention is appropriately determined depending on the age of the patient, the severity of the condition, the route of administration, etc. However, the dose is, e.g. in the range of 0.1-1000 mg, preferably 10-500 mg, more preferably 50-400 mg daily per adult. This dose may be administered once or divided into several portions.

The following examples further illustrate the present invention, but it should be understood that the invention are not limited to these examples.

### EXAMPLES

### Test example 1: Ameliorating effect of a compound of the present invention on the vascular tone-regulating function of vascular endothelium damaged by oxidized LDL (1)

The ability of a compound of the present invention to ameliorate the vascular tone-regulating function of vascular endothelium was evaluated by assaying vascular endothelium-dependent relaxation response to acetylcholine in the presence of a compound of the present invention using cross-section samples of rat vessels damaged by oxidized low-density lipoprotein (oxidized LDL).

Oxidized LDL is a substance derived from an endogenous low-density lipoprotein (LDL) via a exposure to a oxidative stress, and has been reported to be involved in the mechanism by which vascular endothelial cells are damaged (Tanner FC, Noll G, Boulanger CM, Luscher TF, Oxidized low density lipoproteins inhibit relaxations of porcine coronary arteries. Role of scavenger receptor and endothelium-derived nitric oxide. Circulation. 1991, 83:2012-20). The oxidized LDL used in this test was prepared by incubating rabbit LDL suspended at 200 µg/mL in phosphate buffered physiological saline (PBS) prepared by the method of Havel et al. (Havel R.J. et al., J.Clin. Invest., 34, 1345 (1955)) with 5 µM CuSO₄ at 37°C for 24 hours.

First, the mesenteric artery was collected from a rat euthanized by carbon dioxide inhalation into Krebs buffer (containing 118.0 mM NaCl, 4.7 mM KCl, 25.0 mM NaCO₃, 22.5 mM CaCl, 1.5 mM MgSO₄ and 10 mM glucose), and the artery was cut into 2 mm wide annular cross sections. Then, the sections were suspended in a Magnus bath where they were equilibrated under a tension of 3 mN for 60 minutes. After equilibration, the sections were contracted with a 5 µM phenylephrine solution in Krebs buffer.

After confirming that a stable contraction was achieved, the perfusion solution was replaced with Krebs buffer containing 20 µM each of test compounds. The test compounds were the compound of the present invention, 4,6-di-t-butyl-5-hydroxy-2,2-di-n-pentyl-2,3-dihydrobenzofuran, and comparative compounds, probucol and α-tocopherol. Probucol is a compound reported to ameliorate the vascular tone-regulating function of vascular endothelium by preventing the decrease of the EDRF-secreting ability of vascular endothelial cells. α-tocopherol is a fat-soluble endogenous antioxidant known to inhibit the oxidation of LDL.

After perfusion for 30 minutes, oxidized LDL was added to a concentration of 100 µg/mL. After equilibration in the perfusion solution containing oxidized LDL for 60 minutes, acetylcholine (ACh) was cumulatively added at concentrations from 1 x 10⁻¹⁰ M to 1 x 10⁻⁵ M. At equally spaced ACh concentrations, the length of each section was measured to calculate the degree of relaxation (%) by dividing the difference between the lengths of the section before and after relaxation by the length before relaxation. This relaxation is a phenomenon resulting from the acetylcholine-induced secretion of EDRF from vascular endothelial cells.

In addition to the experiment described above, a similar experiment was performed except that the perfusion in Krebs buffer containing test compounds and the equilibration with oxidized LDL were omitted (control experiment) or except that the perfusion in Krebs buffer containing test compounds was omitted but the equilibration with oxidized LDL was included (oxidized LDL experiment).

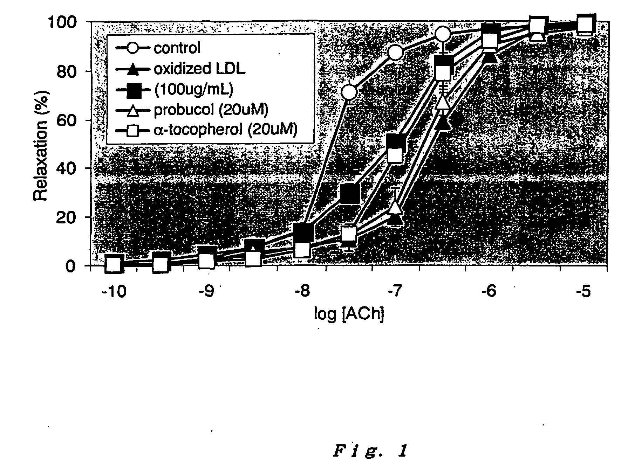

As shown in Figure 1, it can be said that the compound of the present invention ameliorated the vascular tone-regulating function of vascular endothelium, because it achieves relaxation degrees comparable to those of the oxidized LDL experiment at ACh concentrations lower than those of the oxidized LDL experiment. For example, the relaxation degree of 50% was achieved with the compound of the present invention at 126 nM ACh, as compared with 345 nM ACh in oxidized LDL experiment. For reference, the relaxation degree of 50% was achieved at ACh concentrations of 28 nM in the control experiment, 121 nM with probucol, and 261 nM with α-tocopherol.

On the other hand, it can be said that α-tocopherol does not ameliorate the vascular tone-regulating function of vascular endothelium in the ACh concentration range used in this experiment, because it shows only relaxation degrees comparable to those of the oxidized LDL experiment. This means that even α-tocopherol having the ability to inhibit oxidation of LDL cannot inhibit damages to vascular endothelial cells by oxidized LDL or cannot ameliorate the vascular tone-regulating function impaired by such damages. This supports that the ameliorating effect of the compound of the present invention on the vascular tone-regulating function actually inhibited the oxidized LDL-induced impairment of the vascular tone-regulating function of vascular endothelium rather than mere superficial amelioration of the vascular tone-regulating function of vascular endothelium as a result of the removal of a vascular endothelial cell-damaging action from oxidized LDL by inhibiting the oxidation of LDL.

### Test example 2: Ameliorating effect of a compound of the present invention on the vascular tone-regulating function of vascular endothelium damaged by oxidized LDL (2)

The procedure of test example 1 was followed except that the concentration of the test compound was increased from 20 µM to 50 µM.

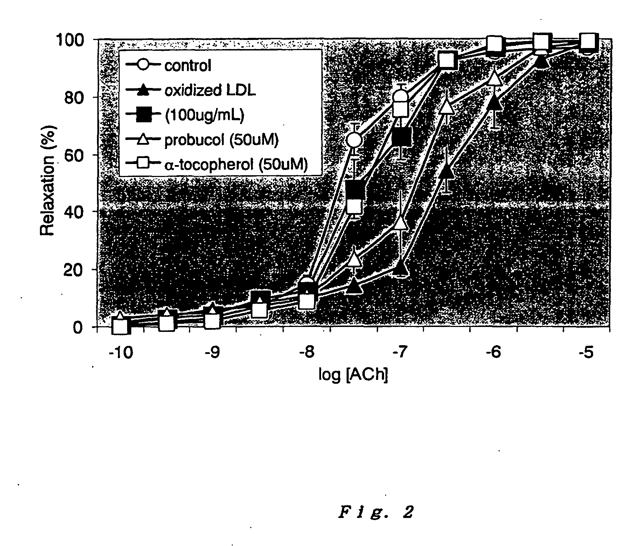

As shown in Figure 2, the compound of the present invention achieves higher relaxation degrees at lower ACh concentrations than in test example 1 by increasing the concentration in solution. Especially, the relation between ACh concentration and relaxation degree closely resembles that of the control, meaning that the adverse influence of oxidized LDL-induced damages to vascular endothelial cells on the vascular tone-regulating function was completely removed.

### INDUSTRIAL APPLICABILITY

The compounds of the present invention are very useful for ameliorating a condition, such as a risk of developing myocardial infarction or intermittent claudication, which is associated with a disease of adults, such as, hypertension, diabetes, arteriosclerosis, or arteriosclerosis obliterans, and in which vascular tone-regulating function is impaired, because the compounds are safer than probucol which has been reported to prevent the decrease of the EDRF-secreting ability of vascular endothelial cells.

## Claims

1. A pharmaceutical composition for ameliorating the vascular tone-regulating function of the vascular endothelium, comprising a compound represented by formula (1): 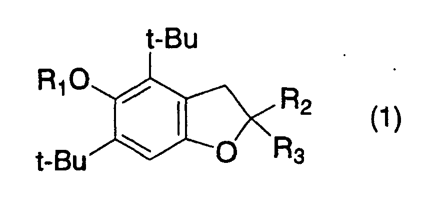 wherein
R₁ represents a hydrogen atom or an acyl or arylalkyloxycarbonyl group;
R₂ and R₃ are identical or different and represent an optionally substituted alkyl group, an optionally substituted alkenyl group or an optionally substituted alkynyl group; or
R₂ and R₃ may together form a cycloalkyl group;
or a pharmaceutically acceptable salt thereof as an active ingredient.

2. The composition of claim 1 wherein R₁ represents a hydrogen atom.

3. The composition of claim 2 wherein R₂ and R₃ represent an optionally substituted alkyl group.

4. The composition of claim 3 wherein the optionally substituted alkyl group is n-butyl, n-pentyl, n-hexyl or n-heptyl group.

5. The composition of claim 1 wherein the compound represented by formula (1) is 4,6-di-t-butyl-5-hydroxy-2,2-di-n-pentyl-2,3-dihydrobenzofuran.

6. The composition of claim 1 wherein a condition in which the amelioration of the vascular tone-regulating function of vascular endothelium is needed is induced by a disease selected from the group consisting of hypertension, diabetes and arteriosclerosis obliterans.

7. The composition of claim 6 wherein the condition in which the amelioration of the vascular tone-regulating function of vascular endothelium is needed is intermittent claudication.

8. A method for ameliorating the vascular tone-regulating function of vascular endothelium, comprising administering to a patient in need thereof, an effective amount of a compound represented by formula (1): 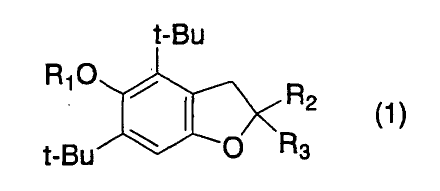 wherein
R₁ represents a hydrogen atom or an acyl or arylalkyloxycarbonyl group;
R₂ and R₃ are identical or different and represent an optionally substituted alkyl group, an optionally substituted alkenyl group or an optionally substituted alkynyl group; or
R₂ and R₃ may together form a cycloalkyl group;
or a pharmaceutically acceptable salt thereof.

9. The method of claim 8 wherein R₁ represents a hydrogen atom.

10. The method of claim 9 wherein R₂ and R₃ represent an optionally substituted alkyl group.

11. The method of claim 10 wherein the optionally substituted alkyl group is n-butyl, n-pentyl, n-hexyl or n-heptyl group.

12. The method of claim 8 wherein the compound represented by formula (1) is 4,6-di-t-butyl-5-hydroxy-2,2-di-n-pentyl-2,3-dihydrobenzofuran.

13. The method of claim 8 wherein a condition in which the amelioration of the vascular tone-regulating function of vascular endothelium is needed is induced by a disease selected from the group consisting of hypertension, diabetes and arteriosclerosis obliterans.

14. The method of claim 13 wherein the condition in which the amelioration of the vascular tone-regulating function of vascular endothelium is needed is intermittent claudication.

15. A use of a compound represented by formula (1): 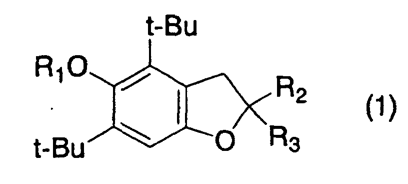 wherein
R₁ represents a hydrogen atom or an acyl or arylalkyloxycarbonyl group;
R₂ and R₃ are identical or different and represent an optionally substituted alkyl group, an optionally substituted alkenyl group or an optionally substituted alkynyl group; or
R₂ and R₃ may together form a cycloalkyl group;
or a pharmaceutically acceptable salt thereof in the manufacture of a pharmaceutical composition for ameliorating the vascular tone-regulating function of vascular endothelium.

16. The use of claim 15 wherein R₁ represents a hydrogen atom.

17. The use of claim 15 wherein R₂ and R₃ represent an optionally substituted alkyl group.

18. The use of claim 17 wherein the optionally substituted alkyl group is n-butyl, n-pentyl, n-hexyl or n-heptyl group.

19. The use of claim 15 wherein the compound represented by formula (1) is 4,6-di-t-butyl-5-hydroxy-2,2-di-n-pentyl-2,3-dihydrobenzofuran.

20. The use of claim 15 wherein a condition in which the amelioration of the vascular tone-regulating function of vascular endothelium is needed is induced by a disease selected from the group consisting of hypertension, diabetes and arteriosclerosis obliterans.

21. The use of claim 20 wherein the condition in which the amelioration of the vascular tone-regulating function of vascular endothelium is needed is intermittent claudication.
